(19) Europäisches Patentamt
European Patent Office
Office européen des brevets

(11) EP 1 567 011 B1

(12) **EUROPÄISCHE PATENTSCHRIFT**

(45) Veröffentlichungstag und Bekanntmachung des Hinweises auf die Patenterteilung:
**10.01.2007 Patentblatt 2007/02**

(21) Anmeldenummer: **03782201.2**

(22) Anmeldetag: **14.11.2003**

(51) Int Cl.:
***A01N 43/90*** (2006.01)

(86) Internationale Anmeldenummer:
**PCT/EP2003/012772**

(87) Internationale Veröffentlichungsnummer:
**WO 2004/045282 (03.06.2004 Gazette 2004/23)**

(54) **FUNGIZIDE MISCHUNGEN AUF DER BASIS EINES TRIAZOLOPYRIMIDIN-DERIVATES UND AMIDVERBINDUNGEN**

FUNGICIDAL MIXTURES BASED ON A TRIAZOLOPYRIMIDINE DERIVATIVE AND AMIDE COMPOUNDS

MELANGES FONGICIDES BASES SUR UN DERIVE TRINZOLOPYRIMIDINE ET DES COMPOSES AMIDES

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **15.11.2002 DE 10253588**

(43) Veröffentlichungstag der Anmeldung:
**31.08.2005 Patentblatt 2005/35**

(73) Patentinhaber: **BASF Aktiengesellschaft**
**67056 Ludwigshafen (DE)**

(72) Erfinder:
- **TORMO I BLASCO, Jordi**
**69514 Laudenbach (DE)**
- **GROTE, Thomas**
**67157 Wachenheim (DE)**
- **AMMERMANN, Eberhard**
**64646 Heppenheim (DE)**
- **STIERL, Reinhard**
**67251 Freinsheim (DE)**
- **STRATHMANN, Siegfried**
**67117 Limburgerhof (DE)**
- **SCHÖFL, Ulrich**
**68782 Brühl (DE)**

(56) Entgegenhaltungen:
**EP-A- 0 988 790**
**WO-A-97/10716**
**WO-A-98/46607**
**WO-A-99/31981**
**WO-A-02/056688**
**US-A- 5 593 996**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft fungizide Mischungen, enthaltend

A) das Triazolopyrimidin der Formel I,

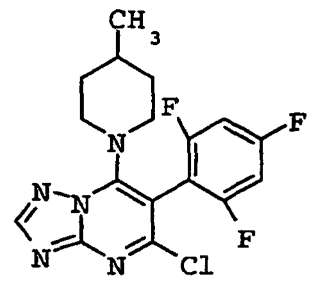

I

und

B) Amidverbindungen der Formel II

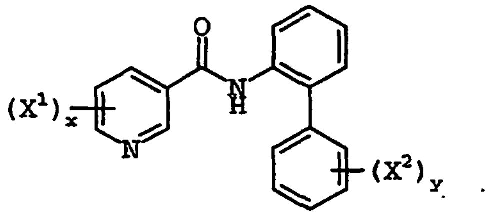

II

worin $X^1$ und $X^2$ gleich oder verschieden sind und für Halogen, Nitro, Cyano, $C_1$-$C_8$-Alkyl, $C_2$-$C_8$-Alkenyl, $C_2$-$C_8$-Alkinyl, $C_1$-$C_8$-Halogenalkyl, $C_2$-$C_8$-Halogenalkenyl, $C_2$-$C_8$-Halogenalkinyl, $C_1$-$C_8$-Alkoxy, $C_1$-$C_8$-Halogenalkoxy, $C_1$-$C_8$-Halogenalkylthio, $C_1$-$C_8$-Alkylsulfinyl oder $C_1$-$C_8$-Alkylsulfonyl stehen;

x 1, 2, 3 oder 4; und

y 1, 2, 3, 4 oder 5 bedeuten;

in einer synergistisch wirksamen Menge.

Außerdem betrifft die Erfindung Verfahren zur Bekämpfung von Schadpilzen mit Mischungen der Verbindungen I und II, sie enthaltende Mittel und die Verwendung der Verbindungen I und II zur Herstellung derartiger Mischungen.

Die Verbindung der Formel I, 5-Chlor-7-(4-methyl-piperidin-1-yl)-6-(2,4,6-trifluor-phenyl-[1,2,4]triazolo[1,5-a] pyrimidin, ihre Herstellung und ihre Wirkung gegen Schadpilze ist aus der Literatur bekannt (WO 98/46607).

In US 5,593,996 wird die Wirksamkeit von Triazolopyrimidinen, wie sie in EP-A 988 790 verwendet werden, gegen pflanzenpathogene Schadpilze wie *Botrytis sp.* gezeigt.

Mischungen von Triazolopyrimidinen mit anderen Wirkstoffen sind aus EP-A 988 790 und US 6,268,371 allgemein bekannt.

Ebenfalls bekannt sind die Amidverbindungen der Formel II, ihre Herstellung und ihre Wirkung gegen Schadpilze (EP-A 545 099).

Mischungen der Amidverbindungen der Formel II mit anderen Wirkstoffen sind ebenfalls bekannt (WO 97/10716, WO 97/39628, WO 99/31981).

WO 02/56688 beschreibt synergistische Mischungen von fungiziden N-Biphenyl-nikotinamiden, wie z.B. Boscalid, mit fungiziden Benzophenonen zur Bekämpfung von pflanzenpathogenen Schadpilzen, wie z.B. *Sphaerotheca sp.*

Der vorliegenden Erfindung lag die Aufgabe zugrunde, weitere besonders wirksame Mischungen zur Bekämpfung von Schadpilzen und insbesondere für bestimmte Indikationen zur Verfügung zu stellen.

Im Hinblick auf eine Senkung der Aufwandmengen und eine Verbesserung des Wirkungsspektrums der bekannten Verbindungen I und II lagen der vorliegenden Erfindung Mischungen als Aufgabe zugrunde, die bei verringerter

Gesamtmenge an ausgebrachten Wirkstoffen eine verbesserte Wirkung gegen Schadpilzen aufweisen (synergistische Mischungen).

Demgemäß wurden die eingangs definierte Mischungen gefunden. Es wurde außerdem gefunden, daß sich bei gleichzeitiger, und zwar gemeinsamer oder getrennter Anwendung der Verbindungen I und der Verbindungen II oder bei Anwendung der Verbindungen I und der Verbindungen II nacheinander Schadpilze besser bekämpfen lassen, als mit den Einzelverbindungen allein.

Die erfindungsgemäßen Mischungen wirken synergistisch und sind daher zur Bekämpfung von Schadpilzen und insbesondere von echten Mehltaupilzen in Getreide, Gemüse, Obst, Zierpflanzen und Reben besonders geeignet.

Die Formel II repräsentiert insbesondere Verbindungen, in denen $X^1$ in 2-Stellung und $X^2$ in 4-Stellung vorliegen (Formel II.1):

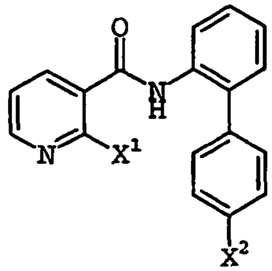

II.1

Bevorzugt sind Verbindungen der Formel II.1, in denen die Kombination der Substituenten einer Zeile der folgenden Tabelle 2 entspricht:

| Nr. | $X^1$ | $X^2$ |
|---|---|---|
| II-1 | F | F |
| II-2 | F | Cl |
| II-3 | F | Br |
| II-4 | Cl | F |
| II-5 | Cl | Cl |
| II-6 | Cl | Br |
| II-7 | $CF_3$ | F |
| II-8 | $CF_3$ | Cl |
| II-9 | $CF_3$ | Br |
| II-10 | $CF_2H$ | F |
| II-11 | $CF_2H$ | Cl |
| II-12 | $CF_2H$ | Br |
| II-13 | $CH_3$ | F |
| II-14 | $CH_3$ | Cl |
| II-15 | $CH_3$ | Br |
| II-16 | $OCH_3$ | F |
| II-17 | $OCH_3$ | Cl |
| II-18 | $OCH_3$ | Br |
| II-19 | $SCH_3$ | F |
| II-20 | $SCH_3$ | Cl |
| II-21 | $SCH_3$ | Br |

(fortgesetzt)

| Nr. | $X^1$ | $X^2$ |
|---|---|---|
| II-22 | S (O) $CH_3$ | F |
| II-23 | S (O) $CH_3$ | Cl |
| II-24 | S(O)$CH_3$ | Br |
| II-25 | $SO_2CH_3$ | F |
| II-26 | $SO_2CH_3$ | Cl |
| II-27 | $SO_2CH_3$ | Br |

Besonders bevorzugt werden die Verbindungen II.1, in denen $X^1$ für $CF_3$ oder Halogen und $X^2$ für Halogen stehen, insbesondere Verbindung II-5 (common name: Boscalid).

Bevorzugt setzt man bei der Bereitstellung der Mischungen die reinen Wirkstoffe I und II, denen man weitere Wirkstoffe gegen Schadpilze oder gegen andere Schädlinge wie Insekten, Spinntiere oder Nematoden oder auch herbizide oder wachstumsregulierende Wirkstoffe oder Düngemittel beimischen kann.

Die Mischungen aus den Verbindungen I und II bzw. die Verbindungen I und II gleichzeitig, gemeinsam oder getrennt angewandt,zeichnen sich durch eine hervorragende Wirkung gegen ein breites Spektrum von pflanzenpathogenen Pilzen, insbesondere aus der Klasse der *Ascomyceten, Deuteromyceten, Phycomyceten* und *Basidiomyceten,* aus. Sie sind z.T. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Bananen, Baumwolle, Gemüsepflanzen (z.B. Gurken, Bohnen und Kürbisgewächse), Gerste, Gras, Hafer, Kaffee, Kartoffeln, Mais, Obstpflanzen, Reis, Roggen, Soja, Tomaten, Wein, Weizen, Zierpflanzen, Zuckerrohr und einer Vielzahl von Samen.

Insbesondere eignen sie sich zur Bekämpfung der folgenden pflanzenpathogenen Pilze: *Blumeria graminis* (echter Mehltau) an Getreide, *Erysiphe cichoracearum* und *Sphaerotheca fuliginea* an Kürbisgewächsen, *Podosphaera leucotricha* an Äpfeln, *Uncinula necator* an Reben, *Puccinia-Arten* an Getreide, *Rhizoctonia*-Arten an Baumwolle, Reis und Rasen, *Ustilago*-Arten an Getreide und Zukkerrohr, *Venturia inaequalis* an Äpfeln, *Bipolaris*- und *Drechslera*-Arten an Getreide, Reis und Rasen, *Septoria nodorum* an Weizen, *Botrytis cinerea* an Erdbeeren, Gemüse, Zierpflanzen und Reben, *Mycosphaerella*-Arten an Bananen, Erdnüssen und Getreide, *Pseudocercosporella herpotrichoides* an Weizen und Gerste, *Pyricularia oryzae* an Reis, *Phytophthora infestans* an Kartoffeln und Tomaten, Pseudoperonospora-Arten an Kürbisgewächsen und Hopfen, *Plasmopara viticola* an Reben, *Alternaria-Arten* an Gemüse und Obst sowie *Fusarium*- und *Verticillium-Arten.*

Sie sind außerdem im Materialschutz (z.B. Holzschutz) anwendbar, beispielsweise gegen *Paecilomyces variotii.*

Die Verbindungen I und II können gleichzeitig, und zwar gemeinsam oder getrennt, oder nacheinander aufgebracht werden, wobei die Reihenfolge bei getrennter Applikation im allgemeinen keine Auswirkung auf den Bekämpfungserfolg hat.

Die Verbindungen I und II werden üblicherweise in einem Gewichtsverhältnis von 100:1 bis 1:100, insbesondere 20:1 bis 1:20, vorzugsweise 20:1 bis 1:5 angewendet.

Die Aufwandmengen der erfindungsgemäßen Mischungen liegen, vor allem bei landwirtschaftlichen Kulturflächen, je nach Art des gewünschten Effekts bei 5 bis 2000 g/ha, vorzugsweise 50 bis 1500 g/ha, insbesondere 50 bis 750 g/ha.

Die Aufwandmengen liegen dabei für die Verbindung I bei 5 bis 2000 g/ha, vorzugsweise 50 bis 1500 g/ha, insbesondere 5 bis 750 g/ha.

Die Aufwandmengen für die Verbindungen II liegen entsprechend bei 5 bis 2000 g/ha, vorzugsweise 50 bis 1500 g/ha, insbesondere 50 bis 750 g/ha.

Bei der Saatgutbehandlung werden im allgemeinen Aufwandmengen an Mischung von 0,001 bis 1 g/kg Saatgut, vorzugsweise 0,01 bis 0,5 g/kg, insbesondere 0,01 bis 0,1 g/kg verwendet.

Sofern für Pflanzen pathogene Schadpilze zu bekämpfen sind, erfolgt die getrennte oder gemeinsame Applikation der Verbindungen I und II oder der Mischungen aus den Verbindungen I und II durch Besprühen oder Bestäuben der Samen, der Pflanzen oder der Böden vor oder nach der Aussaat der Pflanzen oder vor oder nach dem Auflaufen der Pflanzen.

Die erfindungsgemäßen Mischungen, bzw. die Verbindungen I und II können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung

der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:

- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,

- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkyl-arylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpoly-glykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubmittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% der Wirkstoffe. Die Wirkstoffe werden dabei in einer Reinheit von 90% bis 100%, vorzugsweise 95% bis 100% (nach NMR-Spektrum) eingesetzt.

Beispiele für Formulierungen sind:

1. Produkte zur Verdünnung in Wasser

A) Wasserlösliche Konzentrate (SL)

10 Gew.-Teile der Wirkstoffe werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.

B) Dispergierbare Konzentrate (DC)

20 Gew.-Teile der Wirkstoffe werden in Cyclohexanon unter Zusatz eines Dispergiermittels z.B. Polyvinylpyrrolidon gelöst. Bei Verdünnung in Wasser ergibt sich eine Dispersion.

C) Emulgierbare Konzentrate (EC)

15 Gew.-Teile der Wirkstoffe werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

D) Emulsionen (EW, EO)

40 Gew.-Teile der Wirkstoffe werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

E) Suspensionen (SC, OD)

20 Gew.-Teile der Wirkstoffe werden unter Zusatz von Dispergier-und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

F) Wasserdispergierbare und wasserlösliche Granulate (WG, SG)

50 Gew.-Teile der Wirkstoffe werden unter Zusatz von Dispergier-und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

G) Wasserdispergierbare und wasserlösliche Pulver (WP, SP)

75 Gew.-Teile der Wirkstoffe werden unter Zusatz von Dispergier-und Netzmitteln sowie Kieselsäuregel in einer Rotor-Strator Mühle vermahlen. Bei der Verdünnung in Wasser ergibt sich eine stabile Dispersion oder Lösung des Wirkstoffs.

2. Produkte für die Direktapplikation

H) Stäube (DP)

5. Gew.Teile der Wirkstoffe werden fein gemahlen und mit 95 % feinteiligem Kaolin innig vermischt. Man erhält dadurch ein Stäubmittel.

I) Granulate (GR, FG, GG, MG)

0.5 Gew-Teile der Wirkstoffe werden fein gemahlen und mit 95.5 % Trägerstoffe verbunden. Gängige Verfahren sind dabei die Extrusion, die Sprühtrocknung oder die wirbelschicht. Man erhält dadurch ein Granulat für die Direktapplikation.

J) ULV- Lösungen (UL)

10 Gew.-Teile der Wirkstoffe werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubmitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Verbindungen I und II, bzw. die Mischungen oder die entsprechenden Formulierungen werden angewendet, indem man die Schadpilze, die von ihnen freizuhaltenden Pflanzen, Samen, Böden, Flächen, Materialien oder Räume mit einer fungizid wirksamen Menge der Mischung, bzw. der Verbindungen I und II bei getrennter Ausbringung, behandelt. Die Anwendung kann vor oder nach dem Befall durch die Schadpilze erfolgen.

Die fungizide Wirkung der Verbindung und der Mischungen lässt sich durch folgende Versuche zeigen:

Die Wirkstoffe wurden getrennt oder gemeinsam als eine Stammlösung aufbereitet mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf der Basis ethoxylierter Alkylphenole) zugesetzt und entsprechend der gewünschten Konzentration mit Wasser verdünnt.

Anwendungsbeispiel 1 - Kurative Wirksamkeit gegen Weizenbraunrost verursacht durch *Puccinia recondita*

Blätter von in Töpfen gewachsenen Weizensämlingen der Sorte "Kanzler" wurden mit Sporen des Braunrostes (*Puccinia recondita*) bestäubt. Danach wurden die Töpfe für 24 Stunden in eine Kammer mit hoher Luftfeuchtigkeit (90 bis 95 %) und 20 bis 22°C gestellt. Während dieser Zeit keimten die Sporen aus und die Keimschläuche drangen in das Blattgewebe ein. Die infizierten Pflanzen wurden am nächsten Tag mit einer wässriger Suspension in der unten angegebenen Wirkstoffkonzentration bis zur Tropf-Nässe besprüht. Die Suspension oder Emulsion wurde wie oben beschrieben hergestellt. Nach dem Antrocknen des Spritzbelages wurden die Versuchspflanzen im Gewächshaus bei Temperaturen zwischen 20 und 22°C und 65 bis 70 % relativer Luftfeuchte für 7 Tage kultiviert. Dann wurde das Ausmaß der Rostpilzentwicklung auf den Blättern ermittelt.

Die Auswertung erfolgte durch Feststellung der befallenen Blattflächen in Prozent. Diese Prozent-Werte wurden in Wirkungsgrade umgerechnet. Der Wirkungsgrad (W) wurde nach der Formel von Abbot wie folgt bestimmt:

$$W = (1 - \alpha/\beta)\cdot 100$$

α entspricht dem Pilzbefall der behandelten Pflanzen in % und

β entspricht dem Pilzbefall der unbehandelten (Kontroll-) Pflanzen in %

Bei einem Wirkungsgrad von 0 entspricht der Befall der behandelten Pflanzen demjenigen der unbehandelten Kontrollpflanzen; bei einem Wirkungsgrad von 100 wiesen die behandelten Pflanzen keinen Befall auf.

Die zu erwartenden Wirkungsgrade der Wirkstoffmischungen wurden nach der Colby Formel [R.S. Colby, Weeds 15, 20-22 (1967)] ermittelt und mit den beobachteten Wirkungsgraden verglichen.

$$\text{Colby Formel: } E = x + y - x\cdot y/100$$

E zu erwartender Wirkungsgrad, ausgedrückt in % der unbehandelten Kontrolle, beim Einsatz der Mischung aus den Wirkstoffen A und B in den Konzentrationen a und b

x der Wirkungsgrad, ausgedrückt in % der unbehandelten Kontrolle, beim Einsatz des Wirkstoffs A in der Konzentration a

y der Wirkungsgrad, ausgedrückt in % der unbehandelten Kontrolle, beim Einsatz des Wirkstoffs B in der Konzentration b

Tabelle A - Einzelwirkstoffe

| Beispiel | Wirkstoff | Wirkstoffkonzentration in der Spritzbrühe [ppm] | Wirkungsgrad in % der unbehandelten Kontrolle |
|---|---|---|---|
| 1 | Kontrolle (unbehandelt) | - | (90% Befall) |
| 2 | I | 4<br>1 | 56<br>0 |

(fortgesetzt)

| Beispiel | Wirkstoff | Wirkstoffkonzentration in der Spritzbrühe [ppm] | Wirkungsgrad in % der unbehandelten Kontrolle |
|---|---|---|---|
| 3 | II-5 (Boscalid) | 4<br>0,25 | 0<br>0 |

Tabelle B - erfindungsgemäße Mischungen

| Beispiel | Wirkstoffmischung Konzentration Mischungsverhältnis | beobachteter Wirkungsgrad | berechneter Wirkungsgrad*) |
|---|---|---|---|
| 4 | I + II-5 4 + 0,25 ppm 16:1 | 92 | 56 |
| 5 | I + II-5 4 + 4 ppm 1:1 | 92 | 56 |
| 6 | I + II-5 1+4ppm 1:4 | 33 | 0 |
| *) berechneter Wirkungsgrad nach der Colby-Formel | | | |

Anwendungsbeispiel 2 - Wirksamkeit gegen den Grauschimmel an Paprikablättern verursacht durch *Botrytis cinerea* bei protektiver Anwendung

Paprikasämlinge der Sorte "Neusiedler Ideal Elite" wurden, nachdem sich 4 - 5 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1,7 x $10^6$ Sporen/ml in einer 2 %igen wässrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefalls auf den Blättern visuell in % ermittelt werden.

Die Auswertung erfolgte analog Beispiel 1.

Tabelle C - Einzelwirkstoffe

| Beispiel | Wirkstoff | Wirkstoffkonzentration in der Spritzbrühe [ppm] | Wirkungsgrad in % der unbehandelten Kontrolle |
|---|---|---|---|
| 7 | Kontrolle (unbehandelt) | | (95% Befall) |
| 8 | I | 4<br>1<br>0,25 | 68<br>37<br>0 |
| 9 | II-5 (Boscalid) | 1<br>0,25 | 0<br>0 |

Tabelle D - erfindungsgemäße Mischungen

| Beispiel | Wirkstoffmischung Konzentration Mischungsverhältnis | beobachteter Wirkungsgrad | berechneter Wirkungsgrad*) |
|---|---|---|---|
| 10 | I + II-5 4 + 0,25 ppm 16:1 | 95 | 68 |
| 11 | I + II-5 1 + 1 ppm 1:1 | 100 | 37 |
| 12 | I + II-5 0,25 + 0,25 ppm 1:1 | 79 | 0 |
| 13 | I + II-5 0,25 + 1 ppm 1:4 | 89 | 0 |
| *) berechneter Wirkungsgrad nach der Colby-Formel | | | |

Aus den Ergebnissen der Versuche geht hervor, dass der beobachtete Wirkungsgrad in allen Mischungsverhältnissen für die erfindungsgemäßen Kombinationen höher ist, als nach der Colby-Formel vorausberechnet.

## Patentansprüche

1. Fungizide Mischungen, enthaltend

A) das Triazolopyrimidin der Formel I,

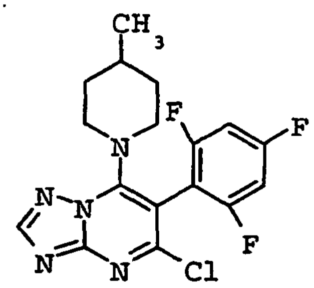

I

und
B) Amidverbindungen der Formel II

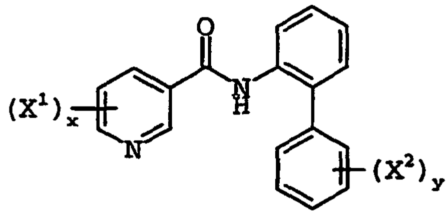

II

worin $X^1$ und $X^2$ gleich oder verschieden sind und für Halogen, Nitro, Cyano, $C_1$-$C_8$-Alkyl, $C_2$-$C_8$-Alkenyl, $C_2$-$C_8$-Alkinyl, $C_1$-$C_8$-Halogenalkyl, $C_2$-$C_8$-Halogenalkenyl, $C_2$-$C_8$-Halogenalkinyl, $C_1$-$C_8$-Alkoxy, $C_1$-$C_8$-Halogenalkoxy, $C_1$-$C_8$-Halogenalkylthio, $C_1$-$C_8$-Alkylsulfinyl oder $C_1$-$C_8$-Alkylsulfonyl stehen;

x 1, 2, 3 oder 4; und
y 1, 2, 3, 4 oder 5 bedeuten;

in einer synergistisch wirksamen Menge.

2. Fungizide Mischungen nach Anspruch 1, wobei die Amidverbindungen der Formel II-1 entsprechen

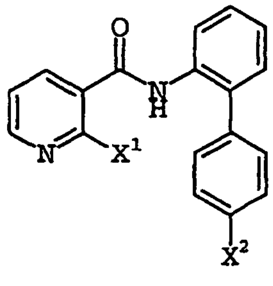

II.1

in der $X^1$ für $CF_3$ oder Halogen und $X^2$ für Halogen steht.

3. Fungizide Mischungen nach Ansprüchen 1 und 2, enthaltend als Amidverbindung der Formel II die Verbindung II-5.

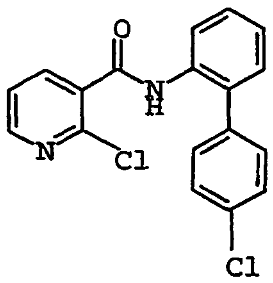

II-5

4. Fungizide Mischungen nach Ansprüchen 1 bis 3, **dadurch gekennzeichnet, daß** das Gewichtsverhältnis des Triazolopyrimidins I zu den Amidverbindungen der Formel II 100:1 bis 1:100 beträgt.

5. Fungizide Mittel, enthaltend die fungiziden Mischungen gemäß Ansprüchen 1 bis 4 sowie einen festen oder flüssigen Träger.

6. Verfahren zur Bekämpfung von pflanzenpathogenen Schadpilzen,
**dadurch gekennzeichnet, daß** man die Schadpilze, deren Lebensraum oder die von ihnen freizuhaltenden Pflanzen, Samen, Böden, Flächen, Materialien oder Räume mit dem Triazolopyrimidin der Formel I gemäß der Ansprüche 1 und 2 und Amidverbindungen der Formel II gemäß der Ansprüche 1 und 3 oder Mitteln gemäß Anspruch 5 behandelt.

7. Verfahren nach Anspruch 6, **dadurch gekennzeichnet, daß** man das Triazolopyrimidin der Formel I gemäß Anspruch 1 und Amidverbindungen der Formel II gemäß Anspruch 1 gleichzeitig, und zwar gemeinsam oder getrennt, oder nacheinander ausbringt.

8. Verfahren nach Anspruch 6 oder 7, **dadurch gekennzeichnet, daß** man das Triazolopyrimidin der Formel I gemäß Anspruch 1 in einer Menge von 5 bis 2000 g/ha aufwendet.

9. Verfahren nach den Ansprüchen 6 oder 7, **dadurch gekennzeichnet, daß** man die Amidverbindungen der Formel II gemäß der Ansprüche 1 und 3 in einer Menge von 5 bis 2000 g/ha aufwendet.

10. Verwendung der Verbindungen I und II gemäß Anspruch 1 zur Herstellung eines zur Bekämpfung von Schadpilzen geeigneten Mittels.

**Claims**

1. A fungicidal mixture, comprising

A) the triazolopyrimidine of the formula I,

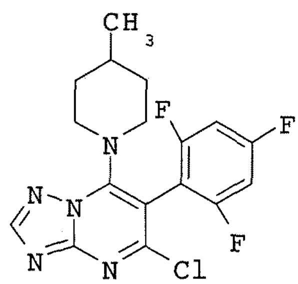

I

and
B) amide compounds of the formula II

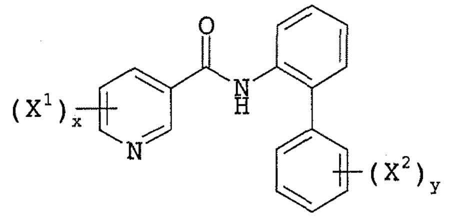

II

where $X^1$ and $X^2$ are identical or different and are halogen, nitro, cyano, $C_1$-$C_8$-alkyl, $C_2$-$C_8$-alkenyl, $C_2$-$C_8$-alkynyl, $C_1$-$C_8$-haloalkyl, $C_2$-$C_8$-haloalkenyl, $C_2$-$C_8$-haloalkynyl, $C_1$-$C_8$-alkoxy, $C_1$-$C_8$-haloalkoxy, $C_1$-$C_8$-haloalkylthio, $C_1$-$C_8$-alkylsulfinyl or $C_1$-$C_8$-alkylsulfonyl;

x is 1, 2, 3 or 4; and
y is 1, 2, 3, 4 or 5;

in a synergistically effective amount.

2. The fungicidal mixture according to claim 1 where the amide compounds correspond to formula II-1

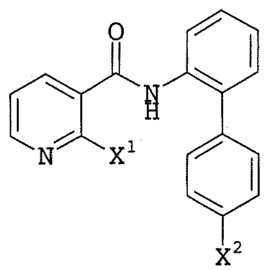

II.1

in which $X^1$ is $CF_3$ or halogen and $X^2$ is halogen.

3. The fungicidal mixture according to claim 1 or 2, comprising, as amide compound of the formula II, the compound II-5.

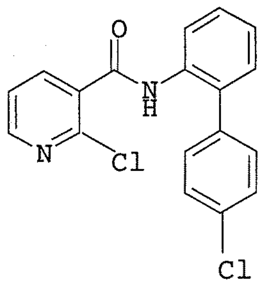

II-5

4. The fungicidal mixture according to any of claims 1 to 3, wherein the weight ratio of the triazolopyrimidine I to the amide compounds of the formula II is from 100:1 to 1:100.

5. A fungicidal composition, comprising the fungicidal mixtures according to any of claims 1 to 4 and a solid or liquid carrier.

6. A method for controlling phytopathogenic harmful fungi, which comprises treating the harmful fungi, their habitat or the plants, seeds, soils, areas, materials or spaces to be kept free from them with the triazolopyrimidine of the formula I according to claim 1 or 2 and amide compounds of the formula II according to claim 1 or 3 or compositions according to claim 5.

7. The method according to claim 6, wherein the triazolopyrimidine of the formula I according to claim 1 and amide

compounds of the formula II according to claim 1 are applied simultaneously, that is either together or separately, or in succession.

**8.** The method according to claim 6 or 7, wherein the triazolopyrimidine of the formula I according to claim 1 is applied in an amount of from 5 to 2000 g/ha.

**9.** The method according to claim 6 or 7, wherein the amide compounds of the formula II according to claim 1 or 3 are applied in an amount of from 5 to 2000 g/ha.

**10.** The use of the compounds I and II according to claim 1 for preparing a composition suitable for controlling harmful fungi.

**Revendications**

**1.** Mélanges fongicides contenant :

A) la triazolopyrimidine de formule I :

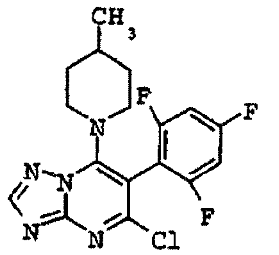

I

et

B) des composés d'amides de formule II :

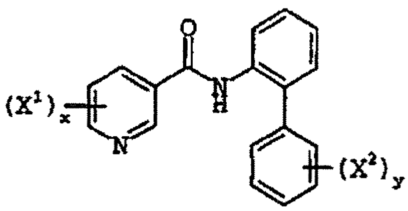

II

dans laquelle $X^1$ et $X^2$ sont identiques ou différents et représentent un halogène ou un groupement nitro, cyano, alkyle en $C_1$-$C_8$, alcényle en $C_2$-$C_8$, alcynyle en $C_2$-$C_8$, halogénoalkyle en $C_1$-$C_8$, halogénoalcényle en $C_2$-$C_8$, halogénoalcynyle en $C_2$-$C_8$, alcoxy en $C_1$-$C_8$, halogénoalcoxy en $C_1$-$C_8$, halogénoalkyl ($C_1$-$C_8$) thio, alkyl ($C_1$-$C_8$) sulfinyle ou alkyl($C_1$-$C_8$)sulfonyle;

x vaut 1, 2, 3 ou 4; et
y vaut 1, 2, 3, 4 ou 5;

en quantité efficace au plan synergique.

**2.** Mélanges fongicides selon la revendication 1, dans lesquels les composés d'amides correspondent à la formule II-1 :

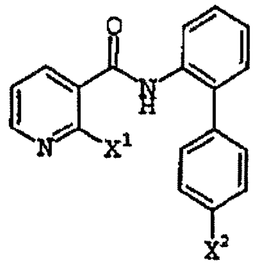

II.1

dans laquelle $X^1$ représente $CF_3$ ou un halogène et $X^2$ représente un halogène.

3. Mélanges fongicides selon les revendications 1 et 2, contenant le composé II-5 comme composé d'amide de formule II :

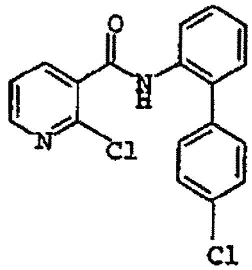

II-5

4. Mélanges fongicides selon les revendications 1 à 3, **caractérisés en ce que** le rapport pondéral de la triazolopyrimidine I aux composés d'amides de formule II est de 100:1 à 1:100.

5. Agents fongicides contenant les mélanges fongicides selon les revendications 1 à 4 ainsi qu'un véhicule solide ou liquide.

6. Procédé pour lutter contre des champignons nuisibles pathogènes des plantes, **caractérisé en ce que** l'on traite les champignons nuisibles, leur biotope ou les plantes, semences, sols, surfaces, matériels ou espaces qui doivent en être protégés par la triazololpyrimidine de formule I selon les revendications 1 et 2 et les composés d'amides de formule II selon les revendications 1 et 3 ou des agents selon la revendication 5.

7. Procédé selon la revendication 6, **caractérisé en ce qu'**on répand la triazolopyrimidine de formule I selon la revendication 1 et des composés d'amides de formule II selon la revendication 1 simultanément, c'est-à-dire conjointement ou séparément, ou successivement.

8. Procédé selon la revendication 6 ou 7,
**caractérisé en ce qu'**on applique la triazolopyrimidine de formule I selon la revendication 1 en quantité de 5 à 2000 g/ha.

9. Procédé selon la revendication 6 ou 7,
**caractérisé en ce que** l'on utilise les composés d'amides de formule II selon les revendications 1 et 3 en quantité de 5 à 2000 g/ha.

10. Utilisation des composés I et II selon la revendication 1 pour fabriquer un agent approprié pour lutter contre les champignons nuisibles.